# EUROPEAN PATENT APPLICATION

(11) **EP 2 206 789 A1**
(43) Date of publication of application: **14.07.2010**
(21) Application number: 10150313.4
(22) Date of filing: 08.01.2010
(51) Int. Cl.: C12Q 1/68, C12P 19/34

(54) **Method for replicating nucleic acid sequence**

(30) Priority: 09.01.2009 JP 2009003420
(71) Applicant: Fujifilm Corporation, Minato-ku Tokyo 106-0031 (JP)
(72) Inventor: Miyoshi, Hayato, Ashigarakami-gun Kanagawa 258-8577 (JP); Iwaki, Yoshihide, Ashigarakami-gun Kanagawa 258-8577 (JP)
(74) Representative: HOFFMANN EITLE

(57) **Abstract**

It is an object of the present invention to provide a method for replicating a nucleic acid sequence using oligonucleotide primers and DNA polymerase. The present invention provides a method for replicating a nucleic acid sequence, which comprises synthesizing a complementary strand with a polymerase that catalyzes a strand displacement complementary strand synthesis reaction, wherein a double-stranded template nucleic acid having a sequence A(Ac) consisting of 20 or more to 200 or less contiguous nucleotides at both ends is used as an origin.

## Description

### Technical Field

The present invention relates to a method for replicating a nucleic acid sequence. More specifically, the present invention relates to a method for replicating a nucleic acid sequence, wherein a polymerase reaction is performed by incubating a reaction solution with DNA polymerase using a template nucleic acid sequence having a specific structure as an origin.

### Background Art

In molecular biological study, the amplification of a nucleic acid has been generally carried out by an emzyamatic method using DNA polymerase. As a method for amplifying a nucleic acid, a polymerase chain reaction (PCR) has been widely known. In order to amplify a target nucleic acid sequence of interest, the PCR method comprises the following three steps: a step of denaturing double-stranded DNA used as a template to convert it to single-stranded DNA (denaturation step); a step of annealing a primer to the single-stranded DNA (annealing step); and a step of elongating a complementary strand using the primer as an origin (elongation step). In an ordinary PCR method, a thermal cycler is used, and the denaturation step, the annealing step and the elongation step are carried out at different temperatures. However, such nucleic acid amplification reaction carried out at the 3 different types of temperatures has required complicated temperature control. Moreover, the PCR method has also been problematic in that time loss increases as the number of cycles increases.

Under the aforementioned circumstances, a nucleic acid amplification method that can be carried out under isothermal conditions has been developed. Examples of such a nucleic acid amplification method include RCA (Rolling Circle Amplification: Proc. Natl. Acad. Sci, Viol. 92, 4641-4645 (1995)), ICAN (Isothermal and Chimeric primer-initiated Amplification of Nucleic acids), LAMP (Loop-Mediated Isothermal Amplification of DNA; Bio Industry, Vol. 18, No. 2 (2001)), NASBA (Nucleic acid Sequence-based Amplification method; Nature, 350, 91- (1991)), and TMA (Transcription mediated amplification method; J. Clin Microbiol. Vol. 31, 3270- (1993)).

The SDA method (JP Patent Publication (Kokai) No. 5-130870 A (1993)) is a cycling assay method using exonuclease, which is one type of amplification method for amplifying a site of interest of a target nucleic acid fragment, utilizing a polymerase elongation reaction. This is a method, which performs a polymerase elongation reaction using, as an origin, a primer specifically hybridizing with such site of interest of a target nucleic acid fragment, and at the same time, allows 5'→3' exonuclease to act thereon, so as to decompose the primer from a reverse direction. Instead of a decomposed primer, a new primer hybridizes with the site, and an elongation reaction with DNA polymerase proceeds again. This elongation reaction with polymerase and the subsequent decomposition reaction with exonuclease for dissociating the elongated strand are successively and periodically repeated. Herein, the elongation reaction with polymerase and the decomposition reaction with exonuclease can be carried out under isothermal conditions. However, in this method, exonuclease as well as polymerase should have been used. Thus, it has required high costs, and further, it has been necessary to device means of designing primers.

The LAMP method is a method for amplifying a site of interest of a target nucleic acid fragment, which has been recently developed. This method uses at least 4 types of primers that complementarily recognize at least 6 specific sites of a target nucleic acid fragment and a strand-displacement-type Bst DNA polymerase that does not have 5'→3' exonuclease activity and catalyzes an elongation reaction while releasing a double-stranded DNA on a template as a single-stranded DNA, so that a site of interest of a target nucleic acid fragment can be amplified as a special structure under isothermal conditions. However, at least 4 types of primers that recognize 6 specific sites should have been used, and thus it has been extremely difficult to design such primers.

The ICAN method is also a method for amplifying a site of interest of a target nucleic acid fragment, which has been recently developed. This is a method for amplifying a gene under isothermal conditions, using an RNA-DNA chimeric primer, DNA polymerase having strand displacement activity and template exchange activity, and RNaseH. After such chimeric primer has bound to a template, a complementary strand is synthesized by DNA polymerase. Thereafter, RNaseH cleaves a chimeric primer-derived RNA portion, and from the cleavage site, an elongation reaction attended with a strand displacement reaction and a template exchange reaction takes place. By repeating this reaction, a gene is amplified. However, this method has also required the use of a special primer that is a chimeric primer, and thus it is extremely difficult to design such primer.

JP Patent Publication (Kohyo) No. 11-509406 A (1999) describes a method for amplifying DNA in a region of interest by reacting it with at least a pair of oligonucleotide primers in the presence of DNA polymerase having strand displacement ability under isothermal conditions. However, the method described in JP Patent Publication (Kohyo) No. 11-509406 A (1999) has been problematic in that it has required a comparatively long reaction time.

JP Patent Publication (Kokai) No. 2002-233379 describes a method for amplifying DNA in a region of interest by reacting it with at least a pair of oligonucleotide primers in the presence of DNA polymerase having strand displacement ability under isothermal conditions. However, the method described in JP Patent Publication (Kokai) No. 2002-233379 has been problematic in terms of a significant level of generation of non-specific amplification products.

### Summary of the Invention

It is an object of the present invention to provide a method for replicating a nucleic acid sequence using oligonucleotide primers and DNA polymerase. It is another object of the present invention to provide a method for replicating a nucleic acid sequence, which can be carried out under isothermal conditions and which enables specific replication of a nucleic acid sequence at a high efficiency in a short time, without requiring complicated primer design or special enzyme

As a result of intensive studies directed towards achieving the aforementioned objects, the present inventors have found that a nucleic acid sequence can be specifically amplified at a high efficiency by providing a nucleic acid sequence having a structure in which a sequence A(Ac) consisting of 20 or more to 200 or less contiguous nucleotides on a template nucleic acid sequence and a sequence B(Bc) consisting of 1 or more to 100 or less nucleotides different from the sequence A(Ac) on the template nucleic acid sequence are alternately arranged, thereby completing the present invention. Ac and Be represent each complimentary sequence ofA and B.

The present invention provides a method for replicating a nucleic acid sequence, which comprises synthesizing a complementary strand with a polymerase that catalyzes a strand displacement complementary strand synthesis reaction, wherein a double-stranded template nucleic acid having a sequence A(Ac) consisting of 20 or more to 200 or less contiguous nucleotides at both ends is used as an origin.

Preferably, the method for replicating a nucleic acid sequence according to the present invention comprises the following steps:
(a) a step of giving a double-stranded template nucleic acid having a structure, in which a sequence A(Ac) consisting of 20 or more to 200 or less contiguous nucleotides and a sequence B(Bc) consisting of 1 or more to 100 or less nucleotides different from the sequence A(Ac) on the template nucleic acid sequence, are alternately arranged, wherein the double-stranded template nucleic acid is characterized in that at least two sequences A(Ac) are present therein;
(b) a step of dissociating the entire or a part of the template nucleic acid given in the step (a);
(c) a step of forming a base pair between a novel nucleic acid strand and the entirely or partially dissociated double-stranded template nucleic acid obtained in the step (b) via the sequences A(Ac); and
(d) a step of synthesizing a complementary strand with a polymerase that catalyzes a strand displacement complementary strand synthesis reaction, wherein the 3'-end(s) of either one or both nucleic acid strands base-paired in the step (c) are used as a synthesis origin(s).

Preferably, the method for replicating a nucleic acid sequence according to the present invention comprises the following steps:
(a) a step of giving a double-stranded template nucleic acid having a structure, in which a sequence A(Ac) consisting of 20 or more to 200 or less contiguous nucleotides and a sequence B(Bc) consisting of 1 or more to 100 or less nucleotides different from tee sequence A(Ac) on the template nucleic acid sequence, are alternately arranged, wherein the double-stranded template nucleic acid is characterized in that at least two sequences A(Ac) are present therein;
(b) a step of dissociating the entire or a part of the template nucleic acid given in the step (a);
(c) a step of forming a base-pair between the entirely or partially dissociated double-stranded template nucleic acids obtained in the step (b) via the sequences A(Ac); and
(d) a step of synthesizing a complementary strand with a polymerase that catalyzes a strand displacement complementary strand synthesis reaction, wherein the 3'-end(s) of either one or both nucleic acid strands base-paired in the step (c) are used as a synthesis origin(s).

Preferably, the reaction solution comprises an oligonucleotide complementary to a part of the double-stranded template nucleic acid.

Preferably, a product from the strand displacement complementary strand synthesis reaction obtained in the step (d) is used as a double-stranded template nucleic acid in the step (a).

Preferably, all the steps are carried out under isothermal conditions.

Preferably, all the steps are carried out at an isothermal temperature between 50°C or higher and 100°C or lower.

Preferably, the polymerase that catalyzes the strand displacement complementary strand synthesis reaction is selected from the group consisting of 5'→3' exonuclease-deficient Bst. DNA polymerase derived from *Bacillus stearothermophilus,* 5'→3' exonuclease-deficient Bca DNA polymerase derived from *Bacillus caldotenax,* 5'→3' exonuclease-deficient Vent. DNA polymerase derived from *Termococcus litoralis,* and DNA polymerase derived from *Alicyclobacillus acidocaldarius.*

Preferably, the reaction solution comprises at least 0.01% or more surfactant.

Preferably, the surfactant is a nonionic surfactant.

Preferably, the reaction solution further comprises a divalent cation.

Preferably, the reaction solution further comprises a melting temperature adjuster.

Preferably, all the steps are carried out within 60 minutes.

According to the present invention, only providing a template nucleic acid sequence having a specific structure and using DNA polymerase having strand displacement activity, such template nucleic acid sequence is continuously elongated. Thus, the template nucleic acid sequences can be specifically replicated at an extremely high efficiency. That is to say, a target nucleic acid sequence can be replicated at a high efficiency in a short time under isothermal conditions, without requiring complicated primer design or special enzyme.

### Brief Description of the Drawings

Figure 1 shows a summary of the method for amplifying a nucleic acid according to the present invention.
Figure 2 shows a summary of the method for amplifying a nucleic acid according to the present invention.
Figure 3 shows a summary of the method for amplifying a nucleic acid according to the present invention.
Figure 4 shows the positional relationship of the primers used in the example with a β2AR gene.
Figure 5 shows the results obtained by the electrophoresis of a product from a replication reaction using primer (1) and primer (3).
Figure 6 shows the results obtained by the electrophoresis of a product from a replication reaction using primer (2) and primer (3).

### Mode for Carrying Out the Invention

Hereinafter, the present invention will be described more in detail.

The method for amplifying a nucleic acid according to the present invention is a method for amplifying a target nucleic acid sequence at a high efficiency in a short time under isothermal conditions, without requiring complicated primer design or special enzyme, which is characterized in that it comprises the following steps:
(a) a step of giving a double-stranded template nucleic acid having a structure, in which a sequence A(Ac) consisting of 20 or more to 200 or less contiguous nucleotides and a sequence B(Bc) consisting of 1 or more to 100 or less nucleotides different from the sequence A(Ac) on the template nucleic acid sequence are alternately arranged, wherein the double-stranded template nucleic acid is characterized in that at least two sequences A(Ac) are present therein;
(b) a step of dissociating the entire or a part of the template nucleic acid given in the step (a);
(c) a step of forming a base pair between a novel nucleic acid strand and the entirely or partially dissociated double-stranded template nucleic acid obtained in the step (b) via the sequences A(Ac); and
(d) a step of synthesizing a complementary strand with a polymerase that catalyzes a strand displacement complementary strand synthesis reaction, wherein the 3'-end(s) of either one or both nucleic acid strands base-paired in the step (c) are used as a synthesis origin(s).

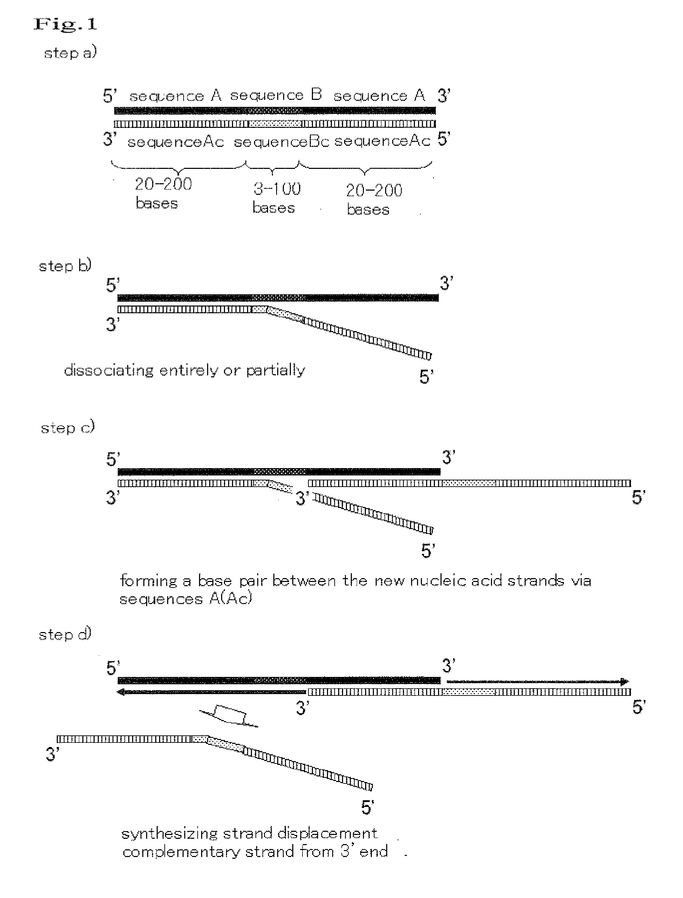

A summary of the method for amplifying a nucleic acid according to the present invention is shown in Figure 1.

According to the present invention the step of dissociating the entire or a part of the template nucleic acid requires neither a special reagent nor a treatment at a high temperature.

Preferably, the step of dissociating the entire or a part of the template nucleic acid is carried out at a temperature between 50°C or higher and 100°C or lower.

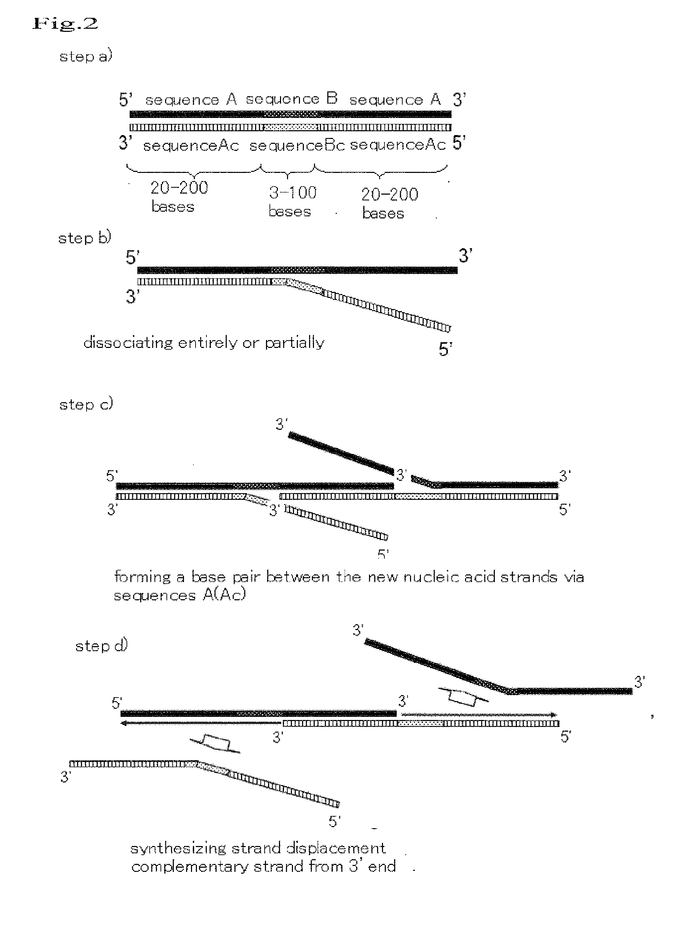

According to the present invention, the novel nucleic acid strand in the step (c) may be provided from an entirely or partially dissociated, different double-stranded template nucleic acid molecule (Figure 2).

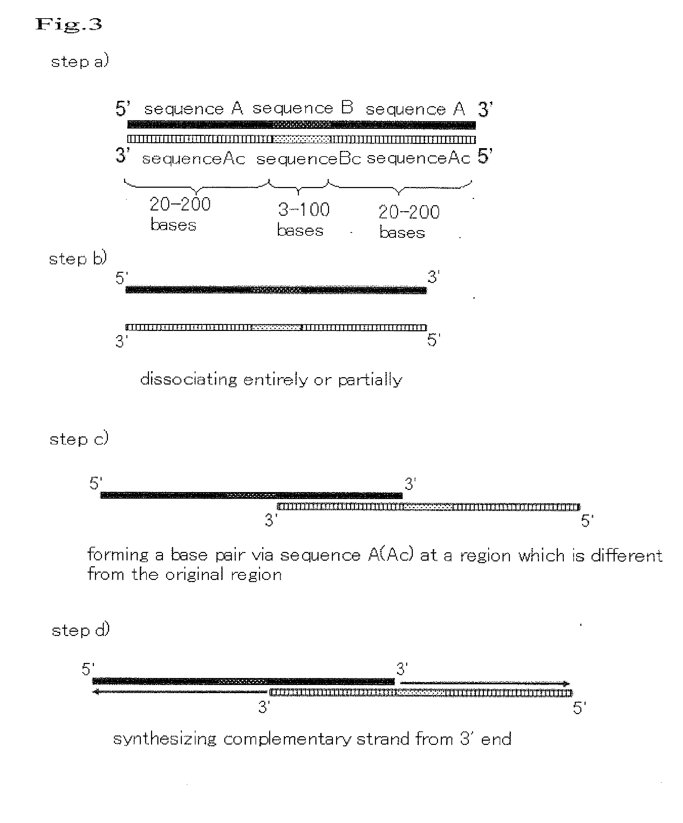

According to the present invention, the novel nucleic acid strand in the step (c) may be provided from an entirely or partially dissociated, original double-stranded template nucleic acid molecule (Figure 3).

According to the present invention, the reaction solution may comprise an oligonucleotide complementary to a part of the double-stranded template nucleic acid.

Hereafter, the ingredients used in the present invention will be described below.

### (1) Deoxynucleotide triphosphate

Deoxynucleotide triphosphate is used as a substrate for an elongation reaction. Specifically, a mixture of dATP, dCTP, dGTP and dTTP is preferably used. Deoxynucleotide triphosphate may comprise analogs of dNTP (for example, 7-deaza-dGTP, etc.).

In addition, the final concentration of such deoxynucleotide triphosphate (a mixture of dATP, dCT1', dGTP and dTTP) is within the range from 0.1 mM to 100 mM, preferably from 0.75 mM to 3.0 mM, more preferably from 1.0 mM to 2.0 mM, and particularly preferably from 1.0 mM to 1.5 mM.

### (2) DNA polymerase

In the present invention, polymerase having strand displacement ability is used. The term "strand displacement ability" is used in the present specification to mean an activity of performing DNA replication using a nucleic acid sequence as a template while displacing a DNA strand, so as to release a complementary strand annealed to a template strand; namely an activity of performing strand displacement. Specific examples of such polymerase having strand displacement ability include 5'→3' exonuclease-deficient Bst. DNA polymerase derived from *Bacillus stearothermophilus,* 5'→3' exonuclease-deficient Bca DNA polymerase derived from *Bacillus caldotenax,* 5'→3' exonuelease-deficient Vent. DNApolymerase derived from *Termococcus litoralis,* and DNA polymerase derived from *Alicyclobacillus acidocaldarius,* but examples are not limited thereto. Such polymerase having strand displacement ability may be either a naturally-occurring protein or a recombinant protein produced by genetic engineering.

### (3) Divalent cation

In the present invention, a divalent cation is used for the metallic requirement of enzyme used, and the like. As such a divalent cation, magnesium salts, calcium salts, and other metal salts can be used. For example, magnesium chloride, magnesium acetate, magnesium sulfate and the like can be used. The final concentration of such a divalent cation is within the range preferably from 1 mM to 20 mM, and more preferably from 2 mM to 10 mM.

### (4) Surfactant

In the present invention, a surfactant may be added into a reaction solution. Using such a surfactant, the advantageous effect of the present invention that is prevention of non-specific amplification of nucleic acids can be achieved. The type of the surfactant used in the present invention is not particularly limited. Examples of the surfactant that can be used in the present invention include: anionic surfactants such as sodium alkylbenzene sulfonate, sodium dodecyl sulfate (SDS), octyl sulfosuccinate or stearic acid soap; nonionic surfactants such as sucrose fatty acid ester, POE sorbitan fatty acid ester (Tween 20, Tween 40, Tween 60, Tween 80, etc.), fatty acid alkanolamide, POE alkyl ether (Brij 35, Brij 58, etc.), POE alkyl phenyl ether (Triton X-100, Triton X-114, Nonidet P40, etc.), nonylphenol, lauryl alcohol, polyethylene glycol, a polyoxyethylene-polyoxypropylene block polymer, POE alkylamine or POE fatty acid bisphenyl ether; and cationic surfactants such as cetyl pyridinium chloride, lauryl dimethyl benzyl ammonium chloride or stearyl trimethyl ammonium chloride. The amount of the surfactant used is not particularly limited, as long as the effect of the present invention can be achieved. The amount of the surfactant used is preferably 0.01% or more, more preferably 0.05% or more, and further preferably 0.1% or more. The upper limit of the amount of the surfactant used is not particularly limited. It is generally 10% or less, preferably 5% or less, and more preferably 1% or less.

Among such surfactants, the use of nonionic surfactants is particularly preferable. Among the nonionic surfactants, a nonionic surfactant with strong hydrophilicity is preferable, and in terms of HLB value, a nonionic surfactant having an HLB value of 12 or greater is preferable. Such an HLB value is more preferably 14 or greater. The upper limit of the HLB value that is preferably applied is 20. The upper limit of the HLB value is more preferably 17 or less, and further preferably from 14 to 17. Structurally, the surfactant used in the present invention is preferably selected from among polyoxyethylene sorbitan fatty acid esters and polyoxyethylene alkyl ethers. Among such polyoxyethylene sorbitan fatty acid esters, those having only one fatty acid ester are preferable. An example of such compound is represented by the following structural formula:

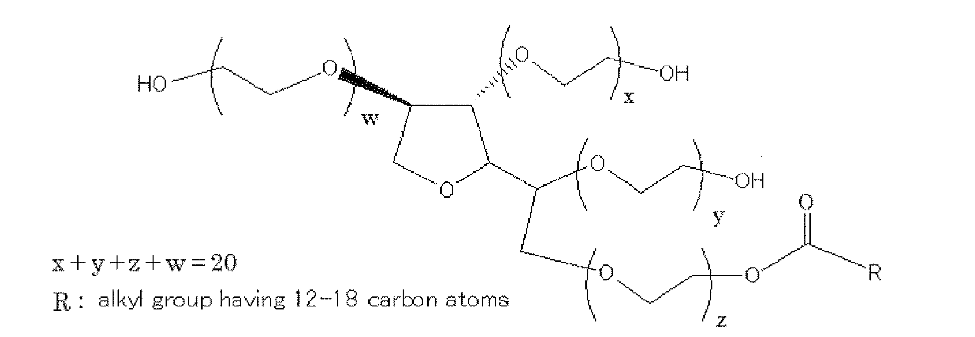

The position of an alkyl group is not particularly limited. The following structures may also be preferably used.

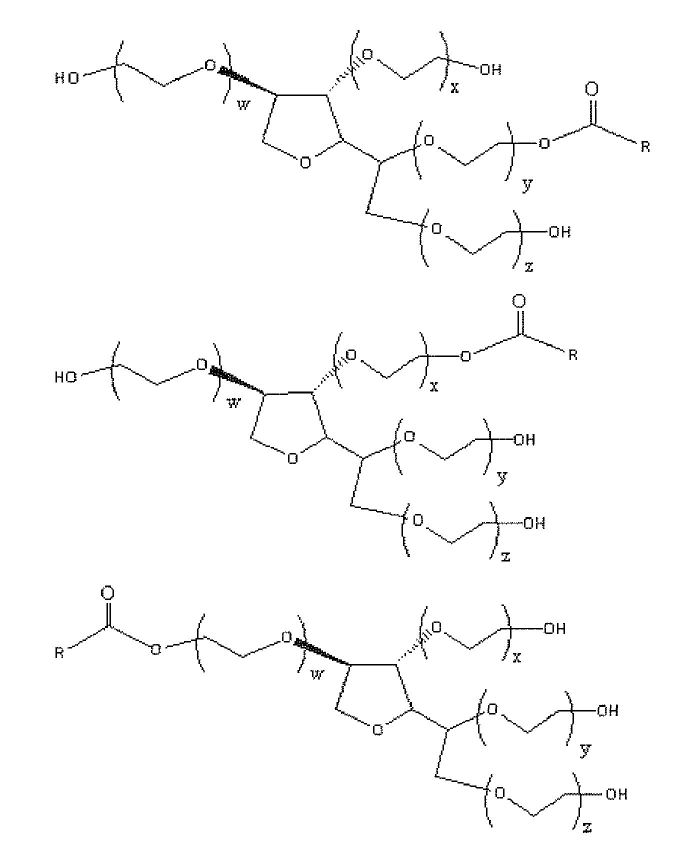

The surfactants represented by the above-descried formulae include nonionic surfactants called polyoxyethylene sorbitan fatty acid esters. Examples of such polyoxyethylene sorbitan fatty acid ester nonionic surfactants include polyoxyethylene (20) sorbitan monolaurate, polyoxyethylene (20) sorbitan monopalmitate, polyoxyethylene (20) sorbitan monostearate, and polyoxyethylene (20) sorbitan monooleate. (Product names: Tween 20, Tween 40, Tween 60, Tween 80, etc.). The amount of such a nonionic surfactant used is not particularly limited. It is preferably 0.01% or more, more preferably 0.05% or more, and further preferably 0.1 % or more.

### (5) Oligonucleotide

The oligonucleotide used in the present invention has a nucleotide sequence substantially complementary to template DNA, and enables the elongation of a DNA strand from the 3'-terminus thereof. Thus, since the oligonucleotide has a nucleotide sequence substantially complementary to template DNA, it can be annealed to DNA used as a template. As the oligonucleotide used in the present invention, an oligonucleotide constituted with deoxyribonucleotide or ribonucleotide may be used, and such oligonucleotide may also comprise a modified ribonucleotide or a modified deoxyribonucleotide.

The length of an oligonucleotide is not particularly limited. The length consists of generally approximately 10 to 100 nucleotides, preferably approximately 15 to 50 nucleotides, and more preferably approximately 15 to 40 nucleotides.

Such an oligonucleotide can be synthesized by a phosphoamidite method using a commercially available DNA synthesizer (for example, Model 394 DNA synthesizer manufactured by Applied Biosystems, etc.).

The amount of such an oligonucleotide used in a reaction solution is preferably 0.1 µM or more, more preferably 1 µM or more, and particularly preferably 1.5 µM or more.

### (6) Melting temperature adjuster

A melting temperature adjuster can be added to the reaction solution in the present invention. Specific examples of such a melting temperature adjuster include dimethyl sulfoxide (DMSO), betaine, formamide, glycerol, tetraalkylammonium salts, and a mixture of two or more types of these compounds. The amount of such a melting temperature adjuster used is not particularly limited. When the melting temperature adjuster is DMSO, formamide, or glycerol, it may be generally contained at a percentage of 10% or less in a reaction solution.

Betaine or tetraalkylammonium salts may be added in a concentration of approximately 0.2 to 3.0 M, and preferably approximately 0.5 to 1.5 M to a reaction solution.

### (7) Buffer component

The reaction solution used in the present invention may comprise a buffer component. The type of such a buffer component is not particularly limited. For example, bicine, tricine, Hepes, Tris, phosphate (sodium phosphate, potassium phosphate, etc.), and the like may be used. The final concentration of a buffer component is within the range from 5 mM to 100 mM, and particularly preferably from 10 mM to 50 mM. The pH value of the buffer component depends on the optimal pH of an enzyme used in an amplification reaction. It is generally from pH 6.0 to 9.0, and particularly preferably from pH 7.0 to 9.0.

### (8) Fluorescent dye

The reaction solution used in the present invention may comprise a fluorescent dye. The type of such fluorescent dye is not particularly limited. For example, SYBR Green I and the like may be used.

Next, the method for amplifying a nucleic acid according to the present invention will be described.

In the present invention, a step of amplifying a nucleic acid can be preferably carried out under substantially isothermal conditions. The temperature for incubation of the reaction solution is preferably 50°C or more, and more preferably 55°C or more. The reaction solution can be incubated around 60°C, for example. The temperature range is preferably from approximately 50°C to approximately 70°C, and more preferably from approximately 55°C to approximately 65°C.

The method for amplifying a nucleic acid according to the present invention can be carried out under substantially isothermal conditions. In the present invention, the term "isothermal conditions" means that each step is carried out at a substantially constant temperature without significantly changing the reaction temperature in each step.

In the present invention, the time required for incubation of the reaction solution under substantially isothermal conditions is not particularly limited, as long as a target nucleic acid sequence can be amplified. The time for incubation is, for example, from 5 minutes to 12 hours. The incubation time is preferably from 5 minutes to 2 hours, more preferably from 5 minutes to 60 minutes, and further preferably from 5 minutes to 30 minutes. It may also be set from 5 minutes to 15 minutes.

A step of amplifying a nucleic acid under substantially isothermal conditions is advantageous in that it is not necessary to increase or decrease the temperature. In the conventional PCR method, it has been necessary to increase or decrease the temperature, and thus a reactor such as a thermal cycler has been required. However, when a step of amplifying a nucleic acid is carried out under substantially isothermal conditions, such step may be carried out using only a device for maintaining a constant temperature.

A method for utilizing the method for amplifying a nucleic acid according to the present invention will be described.

The method for amplifying a nucleic acid according to the present invention can be used in detection of a nucleic acid, labeling, determination of a nucleotide sequence, detection of mutation of nucleotides (including detection of single nucleotide polymorphism and the like), and the like. Since the method for amplifying a nucleic acid of the present invention does not need to use a reaction vessel capable of temperature control, an amplification reaction can be carried out using a large amount of reaction solution.

An amplification product obtained by the method for amplifying a nucleic acid of the present invention can be detected by methods known to persons skilled in the art. For example, according to gel electrophoresis, a reaction product of a specific size can be detected by staining gel with ethidium bromide. As a detection system for detecting an amplification product, fluorescence polarization, immunoassay, fluorescence energy transfer, enzyme labeling (for example, peroxidase, alkaline phosphatase, etc.), fluorescence labeling (for example, fluorescein, rhodamine, etc.), chemiluminescence, bioluminescence, and the like can be used. It is also possible to detect an amplification product using a Taqman probe or Molecular Beacon. It is further possible to detect an amplification product using a labeled nucleotide that is labeled with biotin or the like. In this case, biotin contained in the amplification product can be detected using fluorescently labeled avidin or enzyme-labeled avidin. Moreover, using a redox intercalator known to persons skilled in the art, an amplification product may be detected with electrodes. Furthermore, an amplification product may also be detected using SPR.

By detecting magnesium pyrophosphate, nucleic acid amplification may be detected. In this case, an amplification product may be detected by other methods known to persons skilled in the art, such as detection of turbidity.

The present invention will be more specifically described in the following example. However, these examples are not intended to limit the scope of the present invention.

### EXAMPLES

### <Example 1> Replication of β2AR gene sequence

### (1) Preparation of nucleic acid sample solution containing target nucleic acid sequence

3.0 ng of Human Genomic DNA (manufactured by Clonetech) was heated at 98°C for 3 minutes to prepare a single strand. Thereafter, a sequence in a β2AR gene was amplified under the following conditions.

### <Primers>

The following primers were used so as to obtain, from a sample containing a target nucleic acid sequence, a double-stranded template nucleic acid having a structure in which a sequence A(Ac) consisting of 20 or more to 200 or less contiguous nucleotides and a sequence B(Bc) consisting of 3 or more to 100 or less nucleotides different from the sequence A(Ac) on the template nucleic acid sequence are alternately arranged, wherein the double-stranded template nucleic acid was characterized in that at least two sequences A(Ac) were present therein.

The sequences of individual primers are shown below.
Primer (1):
   5'-CCACGACGCTTGCTGGCACCCAATA3' (SEQ ID NO: 1)
Primer (2):
   5'-TGGGTGGTCTTGCTGGCACCCAATA-3' (SEQ ID NO: 2)
Primer (3);
   5'-CCGGCGCATGGCTT-3' (SEQ ID NO: 3)

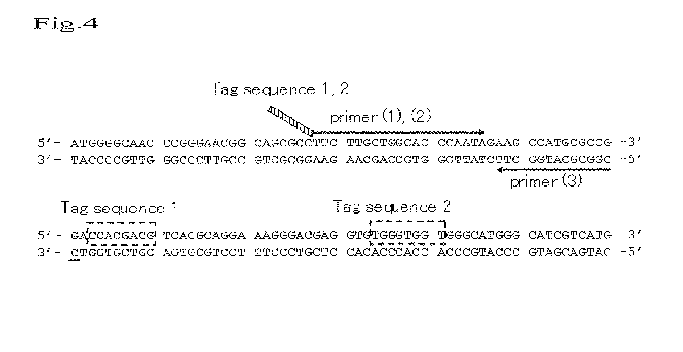

Details of the positional relationship of the aforementioned primers with the β2AR gene are shown in Figure 4.

Herein, 8 nucleotides at the 5'-terminus of each of the primers (1) and (2) are substantially complementary to a sequence existing on the 5'-termina side of a sequence substantially complementary to the primer (3).

### (2) Replication reaction of nucleic acid sequence

An amplification reaction was carried out at 60°C for 60 minutes using a reaction solution with the following composition. As enzyme, Bst. DNA polymerase manufactured by NEB was used.

| <Coxnpositian of reaction solution> | |
|---|---|
| 10 × Bst Buffer (DF) | 1.0 µL |
| 100 mM MgSO₄ | 0.6 µL |
| 10% (v/v) Tween 20 | 0.1 µL |
| 100% DMSO | 0.5 µL |
| 25 mM dNTP each | 0.56 µL |
| SYBR Green I (2000-times diluted) | 0.2 µL |
| 50 µM primer (1) or (2) | 0.64 µL |
| 50 µM primer (3) | 0.64 µL |
| Bst. Polymerase | 0.4 µL |
| A nucleic acid fragment sample obtained in (1) | 3.0 ng |
| Purified water | 4.96 µL |
| | 10.0 µL |

### (3) Detection by electrophoresis

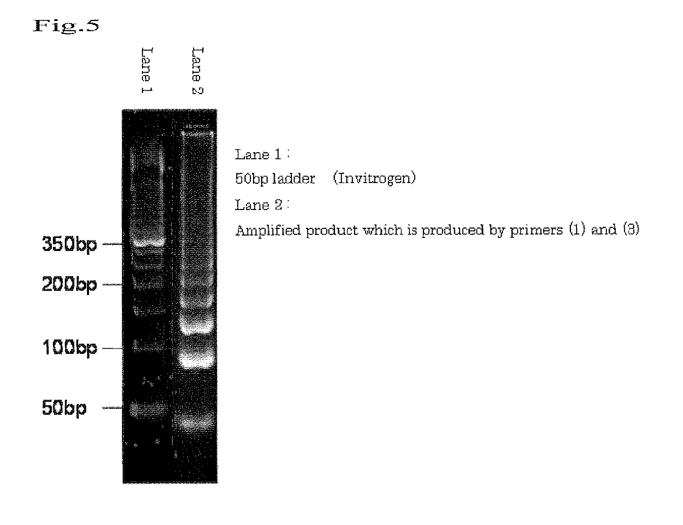

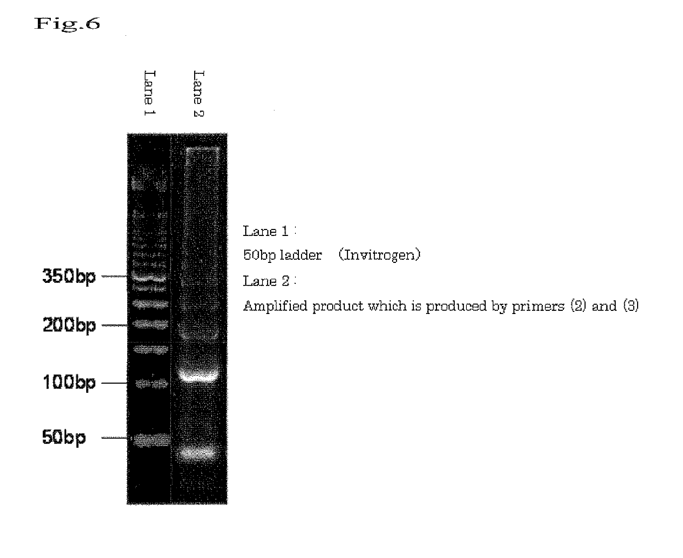

Using 3 wt% agarose gel and a 0.5 × TBE buffer (50 mM Tris, 45 mM Boric acid, 0.5 mM EDTA, pH8.4), electrophoresis was carried out at 100 V for 60 minutes. The results are shown in Figures 5 and 6.

By the combination of the primer (1) with the primer (3), a template nucleic acid sequence consisting of 42 base pairs of sequence A(Ac) and 1 base pair of sequence B(Bc) was obtained. Using, as an origin, a template nucleic acid sequence consisting of 85 base pairs having an A(Ac)B(Bc)A(Ac) structure, by the reaction mechanism shown in Figure 1, Figure 2, or Figure 3, it was assumed that nucleic acid sequences consisting of 128 base pairs, 171 base pairs, 214 base pairs, and 257 base pairs (hereafter, to be polymerized by 43 base pairs) would be replicated. The results shown in Figure 5 corresponded to the predicted band size. Thus, the results demonstrated that a replication reaction of a nucleic acid sequence proceeded with the A(Ac)B(Bc)A(Ac) structure as an origin. A band with the smallest molecular weight was a region (42 base pairs) sandwiched between the primers.

By the combination of the primer (2) with the primer (3), a template nucleic acid sequence consisting of 42 base pairs of sequence A(Ac) and 32 base pairs of sequence B(Bc) was obtained. Using, as an origin, a template nucleic acid sequence consisting of 116 base pairs having an A(Ac)B(Bc)A(Ac) structure, by the reaction mechanism shown in Figure 1 or Figure 2, it was assumed that nucleic acid sequences consisting of 190 base pairs, 264 base pairs, 338 base pairs, and 412 base pairs (hereafter, to be polymerized by 74 base pairs) would be replicated. The results shown in Figure 6 corresponded to the predicted band size. Thus, the results demonstrated that a replication reaction of a nucleic acid sequence proceeded with the A(Ac)B(Bc)A(Ac) structure as an origin. A band with the smallest molecular weight was a region (42 base pairs) sandwiched between the primers.

### (4) Sequencing analysis of replication reaction product

The replication reaction product was purified using NucleoSpin (registered trade mark) Extract II (manufactured by MACHEREY-NAGEL), and then, using TOPO TA Cloning Kit (manufactured by Invitrogen), it was incorporated into a vector. Thereafter, *Escherichia coli* was transformed with the vector. The transformed *Escherichia coli* was cultured in an LB medium containing ampicillin.

Using QIAprep Miniprep (manufactured by Qiagen), plasmid DNA was recovered from the cultured *Escherichia coli.*

The recovered plasmid DNA was sequenced to determine its nucleotide sequence. Such sequencing was carried out using ABI PRISM 310 Genetic Analyzer (manufactured by ABI). As a primer, an M13 Reverse Primer was used.

### M13 Reverse Primer

### 5'-CAGGAAACAGCTATGAC-3' (SEQ ID NO: 4)

As a result of sequencing, it was found that nucleic acids having the following sequences can be obtained by the combination of the primer (1) with the primer (3).

(1)
5'- CCACGACGTT CTTGCTGGCA CCCAATAGAA GCCATGCGCC GG -3'
3'- GGTGCTGCAA GAACGACCGT GGGTTATCTT CGGTACGCGG CC -5'
(42 base pairs) (SEQ ID NO: 5)

(2)
5'- CCACGACGTT CTTGCTGGCA CCCAATAGAA GCCATGCGCC GGACCACGAC -3'
3'- GGTGCTGCAA GAACGACCGT GGGTTATCTT CGGTACGCGG CCTGGTGCTG -5'

5’- GTTCTTGCTG GCACCCAATA GAAGCCATGC GCCGG -3’
3’- CAAGAACGAC CGTGGGTTAT CTTCGGTACG CGGCC -5’
(85 base pairs) (SEQ ID NO: 6)

(3)
5'- CCACGACGTT CTTGCTGGCA CCCAATAGAA GCCATGCGCC GGACCACGAC -3'
3'- GGTGCTGCAA GAACGACCGT GGGTTATCTT CGGTACGCGG CCTGGTGCTG -5'

5'- GTTCTTGCTG GCACCCAATA GAAGCCATGC GCCGGACCAC GACGTTCTTG -3'
3'- CAAGAACGAC CGTGGGTTAT CTTCGGTACG CGGCCTGGTG CTGCAAGAAC -5'

5'- CTGGCACCCA ATAGAAGCCA TGCGCCGG -3'
3'- GACCGTGGGT TATCTTCGGT ACGCGGCC -5'
(128 base pairs) (SEQ ID NO: 7)

As a result of sequencing analysis, it was found that 85 base pairs of nucleic acid sequence having an A(Ac)B(Bc)A(Ac) structure and 128 base pairs of nucleic acid sequence having an A(Ac)B(Bc)A(Ac)B(Bc)A(Ac) structure were present in the replication reaction product.

From the aforementioned results, it was found that the sequence A(Ac) and the sequence B(Bc) could be specifically replicated at a high efficiency from the template nucleic acid sequence having an A(Ac)B(Bc)A(Ac) structure.

As a result of sequencing, it was found that nucleic acids having the following sequences can be obtained by the combination of the primer (2) with the primer (3).

(1)
5'- TGGGTGGTTT CTTGCTGGCA CCCAATAGAA GCCATGCGCC GG -3'
3'- ACCCACCAAA GAACGACCGT GGGTTATCTT CGGTACGCGG CC -5'
(42 base pairs) (SEQ ID NO: 8)

(2)
5'- TGGGTGGTTT CTTGCTGGCA CCCAATAGAA GCCATGCGCC GGACCACGAC -3'
3'- ACCCACCAAA GAACGACCGT GGGTTATCTT CGGTACGCGG CCTGGTGCTG -5'
5'- GTCACGCAGG AAAGGGACGA GGTGTGGGTG GTTTCTTGCT GGCACCCAAT
3'- CAGTGCGTCC TTTCCCTGCT CCACACCCACCAAAGAACGAC CGTGGGTTA
5'- AGAAGCCATG CGCCGG -3'
3'- TCTTCGGTAC GCGGCC -5'
(116 base pairs) (SEQ ID NO: 9)

(3)
5'- TGGGTGGTTT CTTGCTGGCA CCCAATAGAA GCCATGCGCC GGACCACGAC -3'
3'- ACCCACCAAA GAACGACCGT GGGTTATCTT CGGTACGCGG CCTGGTGCTG -5'
5'- GTCACGCAGG AAAGGGACGA GGTGTGGGTG GTTTCTTGCT GGCACCCAAT -3'
3'- CAGTGCGTCC TTTCCCTGCT CCACACCCAC CAAAGAACGA CCGTGGGTTA -5'
5'- AGAAGCCATG CGCCGGACCA CGACGTCACG CAGGAAAGGG ACGAGGTGTG -3'
3'- TCTTCGGTAC GCGGCCTGGT GCTGCAGTGC GTCCTTTCCC TGCTCCACAC -5'
5'- GGTGGTTTCT TGCTGGCACC CAATAGAAGC CATGCGCCGG -3'
3'- CCACCAAAGA ACGACCGTGG GTTATCTTCG GTACGCGGCC -5'
(190 base pairs) (SEQ ID NO: 10)

As a result of sequencing analysis, it was found that 116 base pairs of nucleic acid sequence having an A(Ac)B(Bc)A(Ac) structure and 190 base pairs of nucleic acid sequence having an A(Ac)B(Bc)A(Ac)B(Bc)A(Ac) structure were present in the replication reaction product.

From the aforementioned results, it was found that the sequence A(Ac) and the sequence B(Bc) could be specifically replicated at a high efficiency from the template nucleic acid sequence having anA(Ac)B(Bc)A(Ac) structure.

## Claims

1. A method for replicating a nucleic acid sequence, which comprises synthesizing a complementary strand with a polymerase that catalyzes a strand displacement complementary strand synthesis reaction, wherein a double-stranded template nucleic acid having a sequence A(Ac) consisting of 20 or more to 200 or less contiguous nucleotides at both ends is used as an origin.

2. The method for replicating a nucleic acid sequence according to claim 1, which comprises the following steps:
(a) a step of giving a double-stranded template nucleic acid having a structure, in which a sequence A(Ac) consisting of 20 or more to 200 or less contiguous nucleotides and a sequence B(Bc) consisting of I or more to 100 or less nucleotides different from the sequence A(Ac) on the template nucleic acid sequence, are alternately arranged, wherein the double-stranded template nucleic acid is **characterized in that** at least two sequences A(Ac) are present therein;
(b) a step of dissociating the entire or a part of the template nucleic acid given in the step (a);
(c) a step of forming a base pair between a novel nucleic acid strand and the entirely or partially dissociated double-stranded template nucleic acid obtained in the step (b) via the sequences A(Ac); and
(d) a step of synthesizing a complementary strand with a polymerase that catalyzes a strand displacement complementary strand synthesis reaction, wherein the 3'-end(s) of either one or both nucleic acid strands base-paired in the step (c) are used as a synthesis origin(s).

3. The method for replicating a nucleic acid sequence according to claim 1, which comprises the following steps:
(a) a step of giving a double-stranded template nucleic acid having a structure, in which a sequence A(Ac) consisting of 20 or more to 200 or less contiguous nucleotides and a sequence B(Bc) consisting of 1 or more to 100 or less nucleotides different from the sequence A(Ac) on the template nucleic acid sequence, are alternately arranged, wherein the double-stranded template nucleic acid is **characterized in that** at least two sequences A(Ac) are present therein;
(b) a step of dissociating the entire or a part of the template nucleic acid given in the step (a);
(c) a step of forming a base-pair between the entirely or partially dissociated double-stranded template nucleic acids obtained in the step (b) via the sequences A(Ac); and
(d) a step of synthesizing a complementary strand with a polymerase that catalyzes a strand displacement complementary strand synthesis reaction, wherein the 3'-end(s) of either one or both nucleic acid strands base-paired in the step (c) are used as a synthesis origin(s).

4. The method according to claim 1, wherein the reaction solution comprises an oligonucleotide complementary to a part of the double-stranded template nucleic acid.

5. The method according to claim 2, wherein a product from the strand displacement complementary strand synthesis reaction obtained in the step (d) is used as a double-stranded template nucleic acid in the step (a).

6. The method according to claim 3, wherein a product from the strand displacement complementary strand synthesis reaction obtained in the step (d) is used as a double-stranded template nucleic acid in the step (a).

7. The method according to claim 1, wherein all the steps are carried out under isothermal conditions.

8. The method according to claim 7, wherein all the steps are carried out at an isothermal temperature between 50°C or higher and 100°C or lower.

9. The method according to claim 1, wherein the polymerase that catalyzes the strand displacement complementary strand synthesis reaction is selected from the group consisting of 5'→3' exonuclease-deficient Bst. DNA polymerase derived from *Bacillus stearothermophilus,* 5'→3' exonuclease-deficient Bca DNA polymerase derived from *Bacillus caldotenax,* 5'→3' exonuclease-deficient Vent. DNA polymerase derived from *Termococcus litoralis,* and DNA polymerase derived from *Alicyclobacillus acidocaldarius.*

10. The method according to claim 1, wherein the reaction solution comprises at least 0.01 % or more surfactant.

11. The method according to claim 1, wherein the surfactant is a nonionic surfactant.

12. The method according to claim 1, wherein the reaction solution further comprises a divalent cation.

13. The method according to claim 1, wherein the reaction solution further comprises a melting temperature adjuster.

14. The method according to claim 1, wherein all the steps are carried out within 60 minutes.


SEQUENCE LISTING
<110> FUJIFILM Corporation
<120> Method for replicating nucleic acid sequence
<130> FA9174A
<160> 10
<210> 1
<211> 25
<212> DNA
<213> Artificial Sequence
<220>
<223> Description of Artificial Sequence: Synthetic DNA
<400> 1
ccacgacgct tgctggcacc caata 25
<210> 2
<211> 25
<212> DNA
<213> Artificial Sequence
<220>
<223> Description of Artificial Sequence: Synthetic DNA
<400> 2
tgggtggtct tgctggcacc caata 25
<210> 3
<211> 14
<212> DNA
<213> Artificial Sequence
<220>
<223> Description of Artificial Sequence: Synthetic DNA
<400> 3
ccggcgcatg gctt 14
<210> 4
<211> 17
<212> DNA
<213> Artificial Sequence
<220>
<223> Description of Artificial Sequence: Synthetic DNA

<400> 4
caggaaacag ctatgac                                17
<210> 5
<211> 42
<212> DNA
<213> Artificial Sequence
<220>
<223> Description of Artificial Sequence: amplification product
<400> 5
ccacgacgtt cttgctggca cccaatagaa gccatgcgcc gg                    42
<210> 6
<211> 85
<212> DNA
<213> Artificial Sequence
<220>
<223> Description of Artificial Sequence: amplification product
<400> 6
ccacgacgtt cttgctggca cccaatagaa gccatgcgcc ggaccacgac gttcttgctg    60
gcacccaata gaagccatgc gccgg                                       85
<210> 7
<211> 128
<212> DNA
<213> Artificial Sequence
<220>
<223> Description of Artificial Sequence: amplification product
<400> 7
ccacgacgtt cttgctggca cccaatagaa gccatgcgcc ggaccacgac gttcttgctg    60
gcacccaata gaagccatgc gccggaccac gacgttcttg ctggcaccca atagaagcca   120
tgcgccgg                                                         128
<210> 8
<211> 42
<212> DNA
<213> Artificial Sequence
<220>
<223> Description of Artificial Sequence: amplification product

<400> 8

tgggtggttt cttgctggca cccaatagaa gccatgcgcc gg 42

<210> 9

<211> 116

<212> DNA

<213> Artificial Sequence

<220>

<223> Description of Artificial Sequence: amplification product

<400> 9

tgggtggttt cttgctggca cccaatagaa gccatgcgcc ggaccacgac gtcacgcagg 60

aaagggacga ggtgtgggtg gtttcttgct ggcacccaat agaagccatg cgccgg 116

<210> 10

<211> 190

<212> DNA

<213> Artificial Sequence

<220>

<223> Description of Artificial Sequence: amplification product

<400> 10

tgggtggttt cttgctggca cccaatagaa gccatgcgcc ggaccacgac gtcacgcagg 60

aaagggacga ggtgtgggtg gtttcttgct ggcacccaat agaagccatg cgccggacca 120

cgacgtcacg caggaaaggg acgaggtgtg ggtggtttct tgctggcacc caatagaagc 180

catgcgccgg 190